# FASCICULE DE BREVET EUROPEEN

(11) **EP 3 784 706 B1**
(45) Date de publication et mention de la délivrance du brevet: **03.04.2024**
(21) Numéro de dépôt: 19720936.4
(22) Date de dépôt: 09.04.2019
(51) Int. Cl.: C08F 265/06, A61K 9/16, B01J 13/14, C08F 2/44

(54) **PRÉPARATION D'ÉMULSION POLYMÉRIQUE ACTIVE**
HERSTELLUNG EINER AKTIVEN POLYMEREMULSION
PREPARATION OF AN ACTIVE POLYMER EMULSION

(30) Priorité: 24.04.2018 FR 1853571
(43) Date de publication de la demande: 03.03.2021
(73) Titulaire: Coatex, 69730 Genay (FR)
(72) Inventeur: CHAMPAGNE, Clémentine, 69300 Caluire et Cuire (FR); PARRENIN, Laurie, 01480 Villeneuve (FR); SUAU, Jean-Marc, 69480 Lucenay (FR)
(74) Mandataire: Balmefrezol, Ludovic Francis Pierre
(86) Numéro de dépôt international: PCT/FR2019/000051
(87) Numéro de publication internationale: WO 2019/207213

(56) Documents cités:
- EP-A1- 1 428 867
- EP-A1- 2 877 506
- US-A1- 2017 267 792

## Description

L'invention concerne une méthode de préparation dans l'eau d'un composite comprenant un polymère (P1) et au moins un composé actif. Cette méthode comprend la préparation d'une émulsion comprenant un copolymère de type HASE et un mélange d'un monomère comprenant au moins une insaturation oléfinique polymérisable, d'au moins un composé actif et d'un composé générateur de radicaux, puis la polymérisation du monomère émulsionné par chauffage à une température supérieure à la température requise pour la réaction de polymérisation. L'invention concerne également le composite préparé ainsi que son utilisation.

Il existe des techniques permettant d'associer des composés actifs à différentes substances, notamment des substances utilisées pour protéger le composé actif ou bien des substances pour contrôler la diffusion ou la libération du composé actif. Il existe notamment des méthodes de préparation dans l'eau de polymères pouvant être associés à des composés actifs pour les protéger ou pour en contrôler la diffusion ou la libération. Les méthodes de l'état de la technique ne permettent pas toujours d'apporter de solution satisfaisante aux problèmes rencontrés.

Notamment, les méthodes de l'état de la technique pour l'encapsulation de composés actifs nécessitent l'utilisation d'importantes quantités de composés tensioactifs. Par ailleurs, les méthodes connues ne permettent pas toujours de contrôler la taille des gouttelettes ou particules de monomère émulsionné ou la taille des particules de polymère préparé. Les méthodes de préparation de polymères en milieu aqueux de l'état de la technique ne permettent pas toujours d'aboutir à des niveaux d'extrait sec élevés.

En effet, il est important de pouvoir disposer de moyens permettant d'éviter la libération d'un composé actif ou bien d'éviter la libération non-contrôlée d'un composé actif. Il est également important de pouvoir disposer de moyens permettant de protéger un composé actif, en particulier de protéger un composé actif sensible à l'oxydation ou à la lumière ou bien à d'autres facteurs extérieurs.

De même, il est important de pouvoir disposer de méthodes permettant d'isoler un composé actif d'un environnement qui pourrait l'altérer voire le détériorer, soit lors de sa préparation, soit lors de sa mise en oeuvre.

Il est également important de pouvoir disposer de moyens permettant de contrôler la libération d'un composé actif, en particulier de contrôler cette libération de manière active ou de manière passive. Il est également important de pouvoir disposer de moyens de contrôle de libération d'un composé actif par un changement de pH, par un changement de force ionique, par application d'une contrainte physique ou par un changement de température.

Par ailleurs, il est important de disposer de méthodes de traitement au moyen d'un composé actif, notamment d'un composé actif qui est protégé ou qui est libéré de manière contrôlée. Notamment, il est important de pouvoir mettre en oeuvre des composés actifs fragiles, par exemple des filtres solaires protégés.

Il est également important de pouvoir disposer de méthodes de préparation de composites comprenant un composé actif lipophile, en particulier un filtre solaire, dans une phase hydrophile, en particulier dans une phase aqueuse.

Le document EP 1 428 867 divulgue des matériaux préparés à partir de compositions de polymères à plusieurs étages qui protègent des principes actifs. Ces matériaux sont des polymères de type coeur-écorce (core-shell) ou encore de type multicouches (multi-staged). Le document EP 2 877 506 décrit également une méthode de préparation comprenant plusieurs étapes de polymérisation. Cette méthode conduit à des polymères à plusieurs étages, donc à des polymères multi-couches dont les compositions monomériques respectives diffèrent. Le document EP 3 230 388 divulgue une dispersion de pigment encapsulé dans un polymère ASE non-hydrophobe. Les produits issus de ces méthodes ne permettent pas d'apporter de solution satisfaisante.

Il existe donc un besoin de disposer de méthodes améliorées de mise en oeuvre de composés actifs associées à des polymères, notamment à des polymères préparés dans l'eau. Il existe donc également un besoin de méthodes de préparation améliorées de telles associations de composés actifs et de polymères préparés dans l'eau.

La méthode selon l'invention permet d' apporter une solution à tout ou partie des problèmes des méthodes de l'état de la technique.

Ainsi, l'invention fournit une méthode de préparation dans l'eau d'un composite comprenant un polymère (P1) et au moins un composé actif, comprenant :
(a) la préparation à une température inférieure à 40°C d'un mélange comprenant de l'eau et au moins un copolymère (P2) de type HASE, totalement ou partiellement neutralisé au moyen d'une base ;
(b) l'addition sous agitation et à une température inférieure à 40°C :
   (b1) d'au moins un monomère comprenant au moins une insaturation oléfinique polymérisable ;
   (b2) d'au moins un composé générateur de radicaux ;
   (b3) d'au moins un composé actif et
(c) la polymérisation sous agitation du monomère (b1) par chauffage à une température supérieure à 50°C.

Le copolymère HASE (P2) mis en oeuvre selon l'invention n'est pas un composé tensioactif. Un composé tensio-actif agit en formant une monocouche à l'interface de deux phases hydrophile et lipophile réduisant la tension de surface ; il forme alors des micelles. Une molécule de composé tensio-actif doit donc comprendre deux parties distinctes : une partie lipophile et une partie hydrophile.

De manière avantageuse, la méthode selon l'invention peut être mise en oeuvre avec des quantités réduites ou très réduites de composé tensioactif (moins de 5 % en poids du poids de l'ensemble des monomères, de préférence moins de 3 % en poids ou moins de 1 % en poids du poids de l'ensemble des monomères), voire en l'absence de composé tensioactif.

Lors de la mise en oeuvre des étapes (a) et (b) de la méthode selon l'invention, des gouttelettes de monomère (b1) et de composé (b3) sont dispersées au moyen du copolymère (P2) de type HASE. Lors de la polymérisation (c), les gouttelettes de polymère (P1) sont également dispersées au moyen du copolymère (P2).

De manière particulièrement avantageuse, les gouttelettes formées lors de la mise en oeuvre de la méthode selon l'invention ont une taille contrôlée. Préférentiellement, les gouttelettes de monomère (b1) et de composé (b3) ou de polymère (P1) ont une taille allant de 30 nm à 50 µm, plus préférentiellement de 30 nm à 500 nm ou de 30 nm à 1 µm. Egalement préférentiellement, les gouttelettes de monomère (b1) et de composé (b3) ou de polymère (P1) ont une taille allant de 50 nm à 50 µm, plus préférentiellement de 50 nm à 500 nm ou de 1 µm à 50 µm. La taille des gouttelettes de monomère (b1) et de composé (b3) ou de polymère (P1) peut également aller de 50 nm à 1 µm ou de 500 nm à 50 µm.

De manière habituelle selon l'invention pour mesurer la distribution de taille des particules de phase lipophile dispersées (d₅₀ en %) au sein de la phase hydrophile, on utilise un appareil Mastersizer 2000 Malvern. Elle correspond à la taille pour laquelle 50 % du volume des particules ont une taille inférieure à cette valeur particulière.

Pour la méthode selon l'invention, l'émulsion de monomère (b1) et de composé (b3) ou de polymère (P1) qui est préparée au moyen du copolymère (P2) de type HASE possède un extrait sec qui peut varier de manière assez large. De préférence, cet extrait sec va de 10 % en poids à 50 % en poids, de préférence de 15 % en poids à 45 % en poids ou de 20 % en poids à 40 % en poids d'émulsion.

Le copolymère (P2) mis en oeuvre par la méthode selon l'invention peut généralement être connu en tant que tel. Ce copolymère (P2) est un polymère de type HASE (Hydrophobically modified Alkali-Soluble Emulsion) ou émulsion polymérique modifiée hydrophobiquement qui est rendue soluble par un traitement basique. Il s'agit d'un copolymère acrylique linéaire ou réticulé comprenant des groupements acides et des groupements hydrophobes. Un tel copolymère de type HASE résulte de la copolymérisation de monomères anioniques, tels que les acides acryliques ou méthacryliques, de monomères non ioniques hydrophobes et de macromonomères associatifs hydrophobes. À faible pH, généralement inférieur à 5, et sans l'addition d'une base, le copolymère acrylique de type HASE est très peu ou pas soluble dans l'eau et est présent sous la forme d'un latex. Lors de l'addition d'une base, les groupements anioniques sont partiellement ou totalement neutralisés et le copolymère se solubilise dans l'eau.

De manière préférée pour la méthode selon l'invention, le polymère (P2) de type HASE est obtenu par réaction de polymérisation :
(a1) d'au moins un monomère anionique comprenant au moins une insaturation oléfinique polymérisable, de préférence un monomère anionique comprenant au moins une insaturation oléfinique polymérisable et au moins une fonction acide carboxylique, de préférence le monomère anionique est choisi parmi l'acide acrylique et l'acide méthacrylique, un sel d'acide acrylique, un sel d'acide méthacrylique,
(a2) d'au moins un ester d'un composé dérivé d'un acide choisi parmi l'acide acrylique, l'acide méthacrylique, l'acide itaconique et l'acide maléique, de préférence l'acide acrylique ou l'acide méthacrylique et
(a3) d'au moins un composé de formule (I) :

   R¹-(OE)ₘ-(OP)ₙ-R² (I)

   dans laquelle :
   - m et n, identiques ou différents, représentent indépendamment 0 ou un nombre entier ou décimal inférieur à 150, m ou n est différent de 0,
   - OE représente indépendamment un groupement CH₂CH₂O,
   - OP représente indépendamment un groupement choisi parmi CH(CH₃)CH₂O et CH₂CH(CH₃)O,
   - R¹ représente un groupement C₆-C₄₀-alkyle, linéaire ou ramifié, de préférence un groupement C₈-C₂₀-alkyle, linéaire ou ramifié et
   - R² représente un groupement comprenant au moins une insaturation oléfinique polymérisable, de préférence un groupement acrylate ou un groupement méthacrylate.

De manière préférée selon l'invention, le monomère (a3) est un composé de formule (I) dans laquelle la somme de m et de n est supérieure ou égale à 5, avantageusement supérieure ou égale à 10, plus avantageusement supérieure ou égale à 15, encore plus avantageusement supérieure ou égale à 20.

La somme de m et de n est avantageusement inférieure ou égale à 150, plus avantageusement inférieure ou égale à 120, encore plus avantageusement inférieure ou égale à 100.

De manière préférée selon l'invention, le monomère (a3) est un composé de formule (I) dans laquelle m est supérieur à n, en particulier m est supérieur à 2n.

De manière préférée selon l'invention, le monomère (a3) est un composé de formule (I) dans laquelle n représente 0 et m varie de 5 à 150, avantageusement de 10 à 120, plus avantageusement de 15 à 100, encore plus avantageusement de 20 à 100.

Lors de la préparation du polymère (P2), outre les comonomères (a1), (a2) et (a3), la réaction de polymérisation de la méthode selon l'invention peut mettre en oeuvre un ou plusieurs autre(s) monomère(s).

De manière préférée, un autre monomère utilisable lors de la préparation du polymère (P2) est un composé (a4) choisi parmi acide 2-acrylamido-2-méthylpropane sulfonique, acide éthoxyméthacrylate sulfonique, méthallyl sulfonate de sodium, styrène sulfonate et leurs sels.

La quantité de monomère (a4) peut varier assez largement. De manière préférée, la réaction de préparation du polymère (P2) met en oeuvre de 0,1 % en poids à 7 % en poids, de préférence de 0,5 % en poids à 5 % en poids, plus préférentiellement de 1 % en poids à 3 % en poids de monomère (a4) par rapport à la quantité totale en poids de monomère.

De manière également préférée, un autre monomère utilisable lors de la préparation du polymère (P2) est un composé réticulant (a5). Selon l'invention, un ou plusieurs un composés réticulants (a5) peuvent être mis en oeuvre.

De manière avantageuse selon l'invention, le monomère (a5) est un composé comprenant au moins deux groupements fonctionnels réactifs, notamment deux insaturations oléfiniques polymérisables. Le monomère (a5) est préférentiellement choisi parmi les monomères comprenant au moins deux insaturations oléfiniques. Plus préférentiellement, le monomère (a5) est un composé comprenant au moins deux insaturations éthyléniques. De tels monomères (a5) sont connus en tant que tels.

Comme monomères (a5), on peut citer des monomères insaturés réticulants, par exemple des monomères aromatiques polyinsaturés comme divinylbenzène, divinyl naphthalène et trivinylbenzène, des monomères alicycliques polyinsaturés par exemple 1,2,4-trivinylcyclohéxane, esters d'acide phthalique difonctionnels comme diallyl phthalate, éthers polyalkényl tels que triallyl pentaérythritol, diallyl pentaérythritol, diallyl sucrose, octaallyl sucrose et triméthylolpropane diallyl éther, esters polyinsaturés de polyalcohols ou de polyacides comme 1,6-hexanediol di(méth)acrylate, tétraméthylène tri(méth)acrylate, allyl acrylate, diallyl itaconate, diallyl fumarate, diallyl maléate, triméthylolpropane tri(méth)acrylate, triméthylolpropane di(méth)acrylate, poly(alkylèneoxy) glycol di(méth)acrylate et polyéthylène glycol di(méth)acrylate, alkylène bisacrylamides comme méthylènebisacrylamide et propylène bisacrylamide, dérivés hydroxy ou carboxy de méthylène bis-acrylamide comme N,N'-bisméthylol méthylène bisacrylamide, polyalkylèneglycol di(méth)acrylates comme éthylèneglycol di(méth)acrylate, diéthylèneglycol di(méth)acrylate, triéthylèneglycol di(méth)acrylate, allyl méthacrylate, éthylène glycol diméthacrylate, butylène glycol diméthacrylate, pentaérythritol di-, tri- et tétra-acrylates, poly(alkylèneoxy)glycol di(méth)acrylates tels polyéthylène glycol diacrylates, bisphénol A diacrylates, butanediol diméthacrylate, 2,2-diméthylpropanediol diméthacrylate, phenylène diacrylate et leurs mélanges.

Comme monomère (a5), on peut également citer un composé de formule (II) : 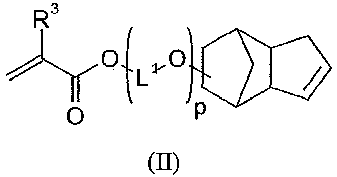 dans laquelle :
- R³ représente indépendamment H ou CH₃,
- L¹ représente indépendamment un groupement C₁-C₂₀-alkylène linéaire ou ramifié et
- p représente indépendamment 0 ou un nombre entier allant de 1 à 30, par exemple de 1 à 20, en particulier de 1 à 15, notamment de 1 à 10.

Comme autre monomère (a5), on peut également citer un composé de formule (III) : 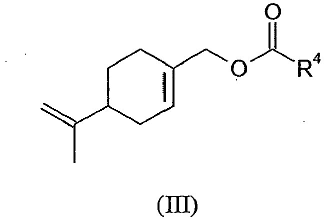 dans laquelle R⁴ représente -C(H)=CH₂, -C(CH₃)=CH₂, -C(H)=C(H)C(O)OH, - C(=CH₂)CH₂C(O)OH, -CH₂C(=CH₂)C(O)OH.

Comme monomère (a5), on peut encore citer un composé de formule (IV) :

H₂C=C(R⁵)CH₂-(OL²)_{q}-O-C(=O)R⁶ (IV)

dans laquelle :
- R⁵ représente indépendamment H ou CH₃,
- R⁶ représente indépendamment -C(H)=CH₂, -C(CH₃)=CH₂, -C(H)=C(H)C(O)OH, -C(H)=C(H)CH₃, -C(=CH₂)CH₂C(O)OH, -CH₂C(=CH₂)C(O)OH,
- L² représente indépendamment un groupement éthylène, propylène ou butylène et
- q représente indépendamment 0 ou un nombre entier ou décimal allant de 1 à 30.

La quantité de monomère (a5) peut également varier assez largement. De manière préférée, la réaction de préparation du polymère (P2) met en oeuvre de 0,01 % en poids à 5 % en poids, de préférence de 0,05 % en poids à 3 % en poids, plus préférentiellement de 0,05 % en poids à 1 % en poids de monomère (a5) par rapport à la quantité totale en poids de monomère.

De manière préférée pour la méthode selon l'invention, le copolymère (P2) de type HASE est partiellement neutralisé au moyen d'une base, par exemple au moyen d'un dérivé de métal alcalin ou d'un dérivé de métal alcalino-terreux. Les bases préférées sont choisies parmi NaOH, KOH, NH₄OH, Ca(OH)₂, monoisopropylamine (AMP), triéthylamine, -diéthylamine, monoéthylamine. De manière préférée selon l'invention, de 15 à 100 % mol des fonctions acides carboxyliques du copolymère (P2) sont neutralisées. De manière plus préférée selon l'invention, de 40 à 100 % mol des fonctions acides carboxyliques du copolymère (P2) sont neutralisées.

La méthode selon l'invention comprend également l'addition sous agitation et à une température inférieure à 40°C d'au moins un monomère (b1) comprenant au moins une insaturation oléfinique polymérisable, d'au moins un composé (b2) générateur de radicaux et d'au moins composé (b3). De manière avantageuse selon l'invention, le comportement dans l'eau du monomère (b1), notamment la solubilité dans l'eau du monomère (b1), permet d'obtenir une émulsion lors du mélange sous agitation dans le copolymère (P2).

Ainsi, on forme une dispersion de gouttelettes, de taille nanométrique à micrométrique, de monomère (b1) et de composé (b3) dans de l'eau.

De manière préférée pour la méthode selon l'invention, le monomère (b1) est un monomère non ionique, de préférence un monomère non ionique comprenant au moins une insaturation oléfinique polymérisable, en particulier une insaturation éthylénique polymérisable et notamment une fonction vinylique polymérisable.

Plus préférentiellement, le monomère (b1) est un monomère non ionique choisi parmi les esters d'un acide comprenant au moins une fonction acide monocarboxylique, en particulier un ester d'un acide choisi parmi l'acide acrylique, l'acide méthacrylique, un sel d'acide acrylique, un sel d'acide méthacrylique et leurs mélanges.

Comme exemples de monomère (b1), on préfère un composé choisi parmi styrène, vinylcaprolactam, N-isopropyl-acrylamide, N-vinyl-pyrrolidone, acrylate d'alkyle, en particulier acrylate de C₁-C₂₄-alkyl, préférentiellement acrylate de C₁-C₂₀-alkyl ou acrylate de C₁-C₄-alkyl, plus préférentiellement acrylate de méthyle, acrylate d'éthyle, acrylate de propyle, acrylate d'isobutyle, acrylate de n-butyle, acrylate de stéaryle, méthacrylate d'alkyle, en particulier méthacrylate de C₁-C₂₄-alkyl, préférentiellement méthacrylate de C₁-C₂₀-alkyl ou méthacrylate de C₁-C₄-alkyl, plus préférentiellement méthacrylate de méthyle, méthacrylate d'éthyle, méthacrylate de propyle, méthacrylate d'isobutyle, méthacrylate de n-butyle, méthacrylate de stéaryle, acrylate d'aryle, de préférence benzylacrylate, phénoxyéthylacrylate, méthacrylate d'aryle, de préférence phénylméthacrylate, benzylméthacrylate, phénoxyéthylméthacrylate. Acrylate de méthyle, acrylate d'éthyle, acrylate de propyle, acrylate d'isobutyle, acrylate de n-butyle, méthacrylate de méthyle, méthacrylate d'éthyle, méthacrylate de propyle, méthacrylate d'isobutyle et méthacrylate de n-butyle sont les monomères (b1) particulièrement préférés.

L'étape (b) selon l'invention met en oeuvre au moins un composé (b2) générateur de radicaux. Il peut être soluble dans l'eau ou bien soluble dans la phase organique, notamment dans le monomère (b1).

De manière préférée pour la méthode selon l'invention, le composé générateur de radicaux (b2), utile comme composé initiateur ou amorceur de la réaction de polymérisation, est un composé azoïque. Plus préférentiellement, le composé générateur de radicaux (b2) est un composé choisi parmi azo-bis-isobutyronitrile (AZDN ou AIBN), un composé peroxyde, de préférence peroxyde de benzoyle, hydroperoxyde de benzoyle et leurs mélanges. On peut également citer les persulfates de métaux alcalins, en particulier le persulfate de sodium et le persulfate de potassium, le persulfate d'ammonium, les peroxydes partiellement hydrosolubles, en particulier l'hydroperoxyde de t-butyle, l'hydroperoxyde de cumyle, les persulfates associés à un ion ferreux, à un ion sulfite ou à un ion bisulfite et leurs mélanges.

Selon l'invention, le composé générateur de radicaux (b2) peut être associé à au moins un agent de transfert de polymérisation radicalaire contrôlée, notamment un agent de transfert de type Raft (*reversible addition-fragmentation chain transfer* ou polymérisation radicalaire contrôlée par transfert de chaîne réversible par addition-fragmentation), par exemple un dérivé de xanthate, de préférence dipropyl trithiocarbonate (DPTTC ou disodium 2,2'-(thiocarbonylbisthio)dipropanoate - n° CAS 864970-33-2), les composés mercaptans, en particulier les composés mercaptans comprenant au moins quatre atomes de carbone tels que le butylmercaptan, le n-octylmercaptan, le n-dodécylmercaptan, le *tert-*dodécylmercaptan, l'isooctyl 3-mercaptopropionate.

De manière préférée lors de la mise en oeuvre de la méthode de préparation selon l'invention, le composé générateur de radicaux (b2), de préférence un composé générateur de radicaux (b2) lipophile, le monomère (b1) et le composé (b3) sont ajoutés sous la forme d'un mélange.

De manière également préférée lors de la mise en oeuvre de la méthode de préparation selon l'invention, le composé générateur de radicaux (b2), de préférence un composé générateur de radicaux (b2) hydrophile, le monomère (b1) et le composé (b3) sont ajoutés séparément ou successivement, de préférence le composé générateur de radicaux (b2) étant alors ajouté après le monomère (b1) et le composé (b3).

Lors de la mise en oeuvre de la méthode de préparation selon l'invention, le composé actif (b3) peut être introduit directement. Il peut également être introduit en mélange avec un autre composé, notamment en mélange avec un solvant. Il peut également être introduit avec le monomère (b1).

Généralement lors de la mise en oeuvre de la méthode de préparation selon l'invention, le composé (b2) générateur de radicaux est introduit séparément du composé actif (b3) et du monomère (b1).

Lors de la réaction de polymérisation du monomère (b1), outre les composés (b1), (b2) et (b3), la réaction de polymérisation de la méthode de préparation selon l'invention peut mettre en oeuvre un ou plusieurs autre(s) monomère(s).

De manière préférée, un autre monomère utilisable lors de la polymérisation du monomère (b1) est un monomère choisi parmi :
(b4) au moins un monomère anionique, de préférence un monomère anionique comprenant au moins une insaturation oléfinique polymérisable, en particulier une insaturation éthylénique polymérisable et notamment une fonction vinylique polymérisable, et au moins une fonction acide carboxylique, de préférence un monomère choisi parmi l'acide acrylique, l'acide méthacrylique, l'acide maléique, l'acide itaconique, l'acide crotonique, un sel d'acide acrylique, un sel d'acide méthacrylique, un sel d'acide maléique, un sel d'acide itaconique, un sel d'acide crotonique,
(b5) au moins un composé choisi parmi acide 2-acrylamido-2-méthylpropane sulfonique, acide éthoxyméthacrylate sulfonique, méthallyl sulfonate de sodium, styrène sulfonate et leurs sels et
(b6) au moins un monomère comprenant au moins deux insaturations oléfiniques,
(b7) au moins un monomère hydrophile non ionique, par exemple vinyl lactams comme N-vinylpyrrolidone, acrylates d'hydroxypropyle, hydroxy-C₁-C₅-alkyl-acrylate, hydroxy-C₁-C₅-alkyl-méthacrylate, acrylamides, méthacrylamides, diacetone-acrylamide, N-isopropylacrylamide.

De manière plus préférée selon l'invention, le monomère (b4) est l'acide méthacrylique. Selon l'invention, la quantité en poids de monomère (b4) mis en oeuvre est inférieure à la quantité en poids de monomère (b1), de préférence bien inférieure à la quantité de monomère (b1). Par exemple, le rapport en poids des monomères (b1) et (b4) peut être supérieur à 3/1, supérieur à 10/1, supérieur à 15/1, supérieur à 20/1 ou encore supérieur à 30/1.

La quantité de monomère (b5) peut varier assez largement. De manière préférée, la réaction de polymérisation du monomère (b1) peut mettre en oeuvre de 0,1 % en poids à 7 % en poids, de préférence de 0,5 % en poids à 5 % en poids, plus préférentiellement de 1 % en poids à 3 % en poids de monomère (b5) par rapport à la quantité totale en poids de monomère.

De manière également préférée, un autre monomère utilisable lors de la polymérisation du monomère (b1) est généralement un composé réticulant (b6).

De manière avantageuse selon l'invention, le monomère (b6) est un composé comprenant au moins deux groupements fonctionnels réactifs, notamment deux insaturations oléfiniques polymérisables. Le monomère (b6) est préférentiellement choisi parmi les monomères comprenant au moins deux insaturations oléfiniques. Plus préférentiellement, le monomère (b6) est un composé comprenant au moins deux insaturations éthyléniques.

De tels monomères (b6) sont connus en tant que tels.

De manière préférée selon l'invention, le monomère (b6) peut être choisi parmi :
- un composé de formule (II) tel que défini pour le monomère (a5) selon l'invention ;
- un composé de formule (III) tel que défini pour le monomère (a5) selon l'invention ;
- un composé de formule (IV) tel que défini pour le monomère (a5) selon l'invention.

Le monomère (b6) peut également être choisi parmi les di(méth)acrylates comme le di(méth)acrylate de polyalkylène glycol, notamment le di(méth)acrylate de polypropylène glycol, le di(méth)acrylate d'éthylène glycol, le di(méth)acrylate de polyéthylène glycol, le di(méth)acrylate de triéthylène glycol, le di(méth)acrylate de 1,3-butylène glycol, le di(méth)acrylate de 1,6-butylène glycol, le di(méth)acrylate de 1,6-hexanediol, le di(méth)acrylate de néopentyl glycol, le di(méth)acrylate de 1,9-nonanediol, mais aussi le 2,2'-bis(4-(acryloxy-propyleoxyphényl))propane, le 2,2'-bis(4-(acryloxydiéthoxy-phényl))propane et l'acrylate de zinc ; les composés tri(méth)acrylates tels que le tri(méth)acrylate de triméthylolpropane et le tri(méth)acrylate de triméthylolpropane éthoxylé, le tri(méth)acrylate triméthyloléthane, le tri(méth)acrylate pentaérythritol et le tri(méth)acrylate de tétraméthylolméthane ; les composés tétra(méth)acrylates tels que le tétra(méth)acrylate de ditriméthylolpropane, le tétra(méth)acrylate de tétraméthylolméthane et le tétra(méth)acrylate de pentaérythritol ; les composés hexa(méth)acrylates tels que l'hexa(méth)acrylate de dipentaérythritol ; les composés penta(méth)acrylates tels que le penta(méth)acrylate de dipentaérythritol ; les composés allyls tels que l'allyl (méth)acrylate, le diallylphthalate, l'itaconate de diallyl, le fumarate de diallyl, le maléate de diallyl ; les éthers polyallyls du sucrose ayant de 2 à 8 groupes par molécule, les éthers polyallyls du pentaérythritol tels que le pentaérythritol diallyl éther, le pentaérythritol triallyl éther et le pentaérythritol tetraallyl éther ; les éthers polyallyls du triméthylolpropane tels que l'éther diallyl triméthylolpropane et l'éther triallyl triméthylolpropane. D'autres composés polyinsaturés incluent le divinyl glycol, le divinyl benzène, le divinylcyclohexyl et le méthylènebisacrylamide.

Le monomère (b6) peut également être préparé par une réaction d'estérification d'un polyol avec un anhydride insaturé tel que l'anhydride acrylique, l'anhydride méthacrylique, l'anhydride maléique ou l'anhydride itaconique. Pour obtenir le monomère (b6), on peut également utiliser des composés choisis parmi les polyhaloalkanols tels que le 1,3-dichloroisopropanol et le 1,3-dibromoisopropanol ; les haloépoxyalkanes tels que l'épichlorohydrine, l'épibromohydrine, le 2-méthyle épichlorohydrine et l'épiiodohydrine ; polyglycidyls éthers tels que le 1,4-butanediol diglycidyl éther, glycérine-1,3-diglycidyl éther, éthylène glycol diglycidyl éther, propylène glycol diglycidyl éther, diéthylène glycol diglycidyl éther, néopentyl glycol diglycidyl éther, polypropylène glycol diglycidyl éther, bisphénol A-épichlorohydrine époxy résine et des mélanges.

Le monomère (b6) peut également être choisi parmi les réticulants trifonctionnels. Il peut s'agir en particulier du tri(méth)acrylate de triméthylolpropane (TMPTA) ou du tri(méth)acrylate de triméthylolpropane éthoxylé (tel que par exemple le TMPTA 3OE).

Le monomère (b6) peut également être choisi parmi le tri(méth)acrylate de triméthylolpropane, tri(méth)acrylate de triméthylolpropane éthoxylé, di(méth)acrylate d'éthylène glycol, méthylènebisacrylamide, diallylphtalate, diallylmaléate et leurs mélanges.

Le monomère (b6) peut également être un mélange de deux monomères distincts, par exemple EGDCPEA (éthylène glycol dicyclopentényl éther acrylate) et TMPTA ou encore EGDCPEA et TMPTA 3OE ou bien encore EGDCPEMA (éthylène glycol dicyclopentényl éther methacrylate) et TMPTA ou EGDCPEMA et TMPTA 3OE.

Selon l'invention, le monomère (b6) est, de préférence, choisi parmi un composé de formule (I), un composé de formule (II), le triméthacrylate de triméthylolpropane, le triacrylate de triméthylolpropane, le triméthacrylate de triméthylolpropane éthoxylé, le triacrylate de triméthylolpropane éthoxylé, le diméthacrylate d'éthylène glycol, le diacrylate d'éthylène glycol, le méthylènebisacrylamide, le diallylphthalate, le diallylmaléate et leurs mélanges.

De manière également avantageuse, la réaction de polymérisation peut mettre en oeuvre moins de 5 % molaire, de préférence de 0,01 à 4 % molaire, en particulier de 0,02 à 4 % molaire ou de 0,02 à 2 % molaire, notamment de 0,02 à 1 % molaire, de monomère (b6) par rapport à la quantité molaire totale de monomères. La réaction de polymérisation peut également mettre en oeuvre de 1 à 4 % molaire ou de 2 à 4 % molaire, de monomère (b6) par rapport à la quantité molaire totale de monomères.

De manière plus préférée selon l'invention, le monomère (b7) est choisi parmi hydroxyéthylacrylate, hydroxyéthylméthacrylate, hydroxypropylacrylate hydroxypropylméthacrylate, N-isopropylacrylamide.

Pour la méthode de préparation selon l'invention, la température des étapes (a) et (b) est inférieure à 40°C afin d'éviter la polymérisation du monomère (b1). De manière avantageuse, la température des étapes (a) et (b), identique ou différente, peut être inférieure à 35°C ou être inférieure à 25°C.

Lors de la mise en oeuvre de la méthode de préparation selon l'invention, le chauffage de l'étape (c) permet de réaliser la polymérisation du monomère (b1). De manière préférée pour la méthode de préparation selon l'invention, la température de l'étape (c) est supérieure à 60°C, de préférence supérieure à 70°C.

Lorsque plusieurs monomères (b1) sont mis en oeuvre, ce chauffage permet de réaliser la copolymérisation de ces monomères (b1). De la même manière, lorsque plusieurs monomères (b1) et (b4) à (b7) sont mis en oeuvre, ce chauffage permet de réaliser la copolymérisation de ces monomères (b1) et (b4) à (b7).

De manière avantageuse, la méthode de préparation selon l'invention peut permettre de préparer une émulsion au sein de laquelle le monomère (b1) et le composé (b3) sont entourés par le copolymère (P2) de type HASE. Le groupement hydrophobe du copolymère (P2) contribue à la formation des gouttelettes. Les gouttelettes de monomère (b1) constituent alors autant de nanoréacteurs ou de microréacteurs de polymérisation.

Lors de la polymérisation du monomère (b1) et de composé (b3) émulsionnés sous forme de gouttelettes entourées de copolymère (P2), le polymère (P1) formé peut alors également être sous la forme de gouttelettes entourées de copolymère (P2). Un composite coeur-écorce peut donc être préparé lors de la mise en oeuvre de la méthode de préparation selon l'invention.

L'invention concerne donc également un composite comprenant au moins un composé actif (b3) et au moins un polymère (P1) préparé selon l'invention, entourés de copolymère (P2) de type HASE mis en oeuvre selon l'invention. Les caractéristiques particulières, avantageuses ou préférées de la méthode de préparation selon l'invention ou du polymère (P1) selon l'invention définissent autant de composites selon l'invention qui sont particuliers, avantageux ou préférés.

En particulier, le composite selon l'invention comprend au moins un polymère (P1), un copolymère (P2) de type HASE et au moins un composé actif (b3), susceptible d'être préparé selon la méthode de préparation selon l'invention.

De manière préférée, le composite selon l'invention comprend un composé actif (b3) qui est encapsulé par du polymère (P1).

Au sein du composite selon l'invention, les quantités relatives de monomère (b1) et de composé actif (b3) peuvent varier assez largement, par exemple en des proportions en poids b1/b3 allant de 99/1 à 5/95. De manière préférée, ces proportions respectives peuvent être de 50/1, de 20/1, de 10/1 ou de 5/1.

Ainsi, l'invention fournit une méthode de protection d'au moins un composé actif par encapsulation au sein d'un composite préparé selon la méthode de préparation définie selon l'invention.

Le composite selon l'invention peut être utilisé dans de nombreux domaines techniques, en particulier selon le choix du composé actif (b3).

De manière préférée, le composé (b3) est un composé hydrophile ou un composé lipophile. Le composé actif (b3) peut également être un composé non-ionique ou un composé apolaire.

De manière plus préférée, le composé (b3) est choisi parmi un composé cosmétique, un composé de soin de la peau, un composé anti-âge, une vitamine, une co-enzyme, un peptide, un composé de soin des cheveux, un composé odorant, un composé aromatique, un composé pharmaceutique, un composé antiseptique, un composé phytosanitaire, un colorant, par exemple phtalocyanine, un pigment, du carbone, par exemple sous forme de nanotubes de carbone, un composé optiquement actif, un composé détergent, un composé adoucissant, un composé luminescent, un composé phosphorescent, un composé fluorescent, un composé métallescent, un composé perlescent, un composé opacifiant, notamment un composé ayant un indice de réfraction supérieur à 1,65 (dioxyde de titane, oxyde de zinc, dioxyde de zinc, alumine, nitrure de bore, talc, kaolin, mica, PbO, ZnSi, ZnSiO₄, ZnS, ZnSe₂), un composé opacifiant revêtu, un composé photochromique, un composé nacré, un composé plastifiant (triethyl citrate, tricaprylin, trilaurin, tripalmitin, triacetin, acetyltriethyl citrate, huile de paraffine), un composé acide gras (acide stearique, acide arachidique, acide palmitoleique, acide oleique, acide linoleique, acide linolaidique, acide arachidonique, acide myristoleique, acide erucique, stéarate de magnésium, oleate de magnésium, stéarate de calcium, linoleate de calcium, stéarate de sodium), un composé huileux, un filtre solaire minéral, un filtre solaire organique, un composé hydratant, un composé azurant optique, un composé blanchissant, un composé antibactérien, le menthol, le chèvrefeuille, l'huile de cananga, le citronellal, l'aurantiol, le limonène, les acides gras, les alcools gras, les beurres, les cires (par exemple les cires d'abeille), les huiles, de préférence une huile choisie parmi les huiles minérales (par exemple l'huile de paraffine, l'huile de vaseline, les huiles minérales ayant un point d'ébullition allant de 300 à 400°C), les huiles d'origine animale (par exemple les squalènes, le squalane, le perhydrosqualène), les huiles végétales (par exemple l'huile d'amande douce, l'huile de calophyllum, l'huile de palme, l'huile de noyau d'abricot, l'huile d'avocat, l'huile de jojoba, l'huile d'olive, l'huile de ricin, les huiles de germes de céréales comme l'huile de germe de blé, la fraction liquide de beurre de karité, l'huile de soja, l'huile de colza), octyl dodecanol, octyldodecyl neopentanoate, triglycéride caprylic/capric, pentaerythrityl tetraisostearate, isodecyl neopentanoate, diisopropyl sebacate, C₁₂-C₁₅ alkyl benzoate, ethylhexyl ethylhexanoate, 2-ethylhexyl hydroxystearate, les composés insaponifiables issus d'huiles naturelles, les huiles synthétiques (par exemple le polyisobutène hydrogéné, les esters d'acides gras tels que l'huile de purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, les esters dérivés d'acide lanolique tels que le lanolate de disopropyle, le lanolate d'isocétyle, les acétylglycérides, les octanoates d'alcool, les octanoates de polyalcools, les décanoates d'alcool, les décanoates de polyalcools, notamment les octanoates de glycol, les octanoates de glycérol, les décanoates de glycol, les décanoates de glycérol, les ricinoléates d'alcools, les ricinoléates de polyalcools), les terpènes, les polyterpènes, le phytostérols, les huiles siliconées (par exemple les cyclométhicones, les polydiméthylsiloxanes de faible poids moléculaire ou huiles de silicone, les polydiméthylsiloxanes de haut poids moléculaire ou gommes de silicone, les polyméthylsiloxanes, les diméthiconols, les polydiméthylsiloxanes phénylées, les siloxanols de faible poids moléculaire, les siloxanols de haut poids moléculaire, les triméthylsiloxysilicates), les huiles fluorées (par exemple les perfluoroéthers fluorés et les silicones fluorés) et leurs combinaisons ; de préférence le composé actif (b3) est choisi parmi les composés actifs cosmétiques, par exemple un composé optiquement actif, un composé de soin de la peau, un composé anti-âge, une vitamine, une co-enzyme, un peptide, un composé de soin des cheveux, un composé odorant, un composé aromatique.

Selon l'invention, le composé actif (b3) est préférentiellement un composé nonpolymérisable. Il est inactif vis-à-vis des monomères mis en oeuvre lors de la réaction de polymérisation.

L'invention fournit également une méthode de traitement ou d'utilisation comprenant l'application d'au moins un composite selon l'invention, de préférence l'application sur un substrat, par exemple un substrat kératinique, en particulier les cheveux, les poils, les ongles et la peau.

Notamment, selon le polymère (P1) mis en oeuvre ou selon le composé actif (b3) utilisé, la méthode de traitement ou d'utilisation selon l'invention peut être de différentes natures.

La méthode de traitement ou d'utilisation selon l'invention peut être une méthode de traitement non-thérapeutique. La méthode de traitement selon l'invention est généralement une méthode de traitement non-thérapeutique. L'invention fournit également une méthode de traitement mettant en oeuvre au moins un composite selon l'invention.

De manière préférée, lors de la mise en oeuvre de la méthode de traitement selon l'invention le composé actif (b3) n'est pas libéré du composite. Le composé (b3) est alors protégé au sein du composite selon l'invention.

De manière également préférée, lors de la mise en oeuvre de la méthode de traitement selon l'invention le composé actif (b3) est libéré. Il peut être libéré de manière active ou libéré de manière passive. De manière également plus préférée, le composé actif (b3) est libéré de manière progressive dans le temps.

De manière bien plus préférée, le composé actif (b3) est libéré au moyen du composite selon l'invention. En particulier, il est libéré de manière contrôlée ou de manière progressive, par exemple libéré par un changement de pH, par un changement de force ionique, par application d'une contrainte physique ou par un changement de température.

### EXEMPLES

Les exemples qui suivent permettent d'illustrer les différents aspects de l'invention.

### Exemple 1 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 *%* en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,0 g d'une composition B comprenant 93,4 % en poids de méthacrylate de méthyle, 6,0 % en poids de pigment (dioxyde de titane traité en surface à l'acide stéarique et à l'hydroxyde d'aluminium Mitsubishi) (b3) et 0,6 % en poids de réticulant diméthacrylate d'éthylène glycol (b6), puis
- 10,85 g d'une solution aqueuse de 4,4'-azobis(4-cyanopentanoic acid) (0,56 g) et 2-amino-2-méthylpropanol (0,29 g) (b2), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 21,5 % en poids et la taille des particules est répartie en deux populations : une majoritaire à 180 nm et une autre à 1 600 nm. Le réacteur ne contient pas de composé actif (b3) surnageant, la dispersion est homogène. Le composé actif (b3) a été encapsulé par le copolymère (P1) en présence du copolymère (P2).

### Exemple 2 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 *%* en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids de filtre solaire 2-ethylhexyl methoxycinnamate (Sigma Aldrich N° CAS 5466-77-3) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 23,6 % en poids et la taille des particules est répartie en deux populations : une majoritaire à 240 nm et une autre à 5 050 nm. Le diamètre d₅₀, pour lequel au moins 50 % en volume des particules ont une taille inférieure à une certaine taille, est de 140 nm. Le réacteur ne contient pas de composé actif (b3) ayant précipité, la dispersion est homogène. Le composé actif (b3) a été encapsulé par le copolymère (P1) en présence du copolymère (P2).

### Exemple 3 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,9 % en poids de méthacrylate de butyle (b1), 4,5 % en poids de méthacrylate de stéaryle (b1), 6,1 % en poids de filtre solaire 2-ethylhexyl methoxycinnamate (Sigma Aldrich N° CAS 5466-77-3) (b3) et 0,5 % en poids de réticulant diméthacrylate d'éthylène glycol (b6), puis
- une solution aqueuse d'acide 4,4'-azo-bis(4-cyanopentanoique) (0,56 g) et de 2-amino-2-méthylpropanol (0,29 g) (b2) dans 5,85 g d'eau, puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 22,7 % en poids et la taille des particules est de 120 nm. Le diamètre d₅₀, pour lequel au moins 50 % en volume des particules ont une taille inférieure à une certaine taille, est de 120 nm. Le réacteur ne contient pas de composé actif (b3) ayant précipité, la dispersion est homogène. Le composé actif (b3) a été encapsulé par le copolymère (P1) en présence du copolymère (P2).

### Exemple 4 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,20 g de 2-amino-2-méthylpropanol ;
- 58,6 g d'une composition B comprenant 88,9 % en poids de méthacrylate de butyle (b1), 4,5 % en poids de méthacrylate de stéaryle (b1), 6,1 % en poids de filtre solaire 2-ethylhexyl methoxycinnamate (Sigma Aldrich N° CAS 5466-77-3) (b3) et 0,5 % en poids de réticulant diméthacrylate d'éthylène glycol (b6), puis
- 10,85 g d'une solution aqueuse d'acide 4,4'-azo-bis(4-cyanopentanoique) (0,56 g) et de 2-amino-2-méthylpropanol (0,29 g) (b2) dans 10 g d'eau, puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 23,5 % en poids et la taille des particules est répartie en deux populations : une majoritaire à 180 nm et une autre à 2 190 nm. Le diamètre d₅₀, pour lequel au moins 50 % en volume des particules ont une taille inférieure à une certaine taille, est de 300 nm. Le réacteur ne contient pas de composé actif (b3) ayant précipité, la dispersion est homogène. Le composé actif (b3) a été encapsulé par le copolymère (P1) en présence du copolymère (P2).

### Exemple 5 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids de parfum Geraniol (Sigma Aldrich N° CAS 106-24-1) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidit à température ambiante.

On obtient une dispersion d'un composite selon l'invention comprenant un polymère (P1), un composé actif (b3) et un copolymère (P2). L'extrait sec final est de 22,2 % en poids et la taille des particules est répartie en deux populations : une minoritaire à 240 nm et une majoritaire à 3 200 nm. Le diamètre d₅₀, pour lequel au moins 50 % en volume des particules ont une taille inférieure à une certaine taille, est de 730 nm. Le réacteur ne contient pas de composé actif (b3) surnageant, la dispersion est homogène. Le composé actif (b3) a été encapsulé par le copolymère (P1) en présence du copolymère (P2).

### Exemple 6 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids d'une huile d'origine végétale (huile de tournesol commerciale alimentaire Eco+) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 24,3 % en poids et la taille des particules est répartie en deux populations : une majoritaire à 140 nm et une autre à 1 450 nm. Le diamètre d₅₀, pour lequel au moins 50 % en volume des particules ont une taille inférieure à une certaine taille, est de 270 nm. Le réacteur ne contient pas de composé actif (b3) surnageant, la dispersion est homogène. Le composé actif (b3) a été encapsulé par le copolymère (P1) en présence du copolymère (P2).

### Exemple 7 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-CiôOEzs (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids d'un pigment inorganique (oxyde de fer rouge Maprecos C33-A8075 SunCroma N° CAS 1309-37-1) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 22,4 % en poids et la taille des particules est répartie en trois populations : une majoritaire à 120 nm, une autre à 720 nm et une dernière à 3 800 nm. Le diamètre d₅₀, pour lequel au moins 50 % en volume des particules ont une taille inférieure à une certaine taille, est de 120 nm. Le réacteur ne contient pas de composé actif (b3) ayant précipité, la dispersion est homogène. Le composé actif (b3) a été encapsulé par le copolymère (P1) en présence du copolymère (P2).

### Exemple 8 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,20 g de 2-amino-2-méthylpropanol ;
- 68,0 g d'une composition B comprenant 63,3 % en poids de méthacrylate de butyle (b1), 15,5 % en poids de méthacrylate de stéaryle (b1), 6,0 % en poids de pigment (oxyde de fer rouge (Maprecos C33-A8075 SunCroma N° CAS 1309-37-1) (b3), 0,5 % en poids de réticulant diméthacrylate d'éthylène glycol (b6) et 14,7 % en poids de N-méthyl-2-pyrrolidone, puis
- 10,85 g d'une solution aqueuse d'acide 4,4'-azo-bis(4-cyanopentanoique) (0,56 g) et 2-amino-2-méthylpropanol (0,29 g) (b2), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 22,7 % en poids et la taille des particules est de 120 nm. Le diamètre d₅₀, pour lequel au moins 50 % en volume des particules ont une taille inférieure à une certaine taille, est de 110 nm. Le réacteur ne contient pas de composé actif (b3) ayant précipité, la dispersion est homogène. Le composé actif (b3) a été encapsulé par le copolymère (P1) en présence du copolymère (P2).

### Exemple 9 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids de pigment (dioxyde de titane traité en surface à l'acide stéarique et à l'hydroxyde d'aluminium Mitsubishi) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 23,7 % en poids et la taille des particules est répartie en deux populations : une majoritaire à 140 nm et une autre à 1 100 nm. Le diamètre d₅₀, pour lequel au moins 50 % en volume des particules ont une taille inférieure à une certaine taille, est de 170 nm. Le réacteur ne contient pas de composé actif (b3) surnageant, la dispersion est homogène. Le composé actif (b3) a été encapsulé par le copolymère (P1) en présence du copolymère (P2).

### Exemple 10 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,0 g d'une composition B comprenant 93,4 % en poids de styrene, 6,0 % en poids du pigment/filtre solaire (dioxyde de titane traité en surface à l'acide stéarique et à l'hydroxyde d'aluminium Mitsubishi) (b3) et 0,6 % en poids de réticulant diméthacrylate d'éthylène glycol (b6), puis
- 10,85 g d'une solution aqueuse de 4,4'-azobis(4-cyanopentanoic acid) (0,56 g) et 2-amino-2-méthylpropanol (0,29 g) (b2), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient un composite selon l'invention comprenant un polymère (P1), un composé actif (b3) et un copolymère (P2). L'extrait sec final est de 21,8 % en poids et la taille des particules est répartie en deux populations : une majoritaire à 140 nm et une autre à 2 880 nm. Le diamètre d₅₀, pour lequel au moins 50 % en volume des particules ont une taille inférieure à une certaine taille, est de 134 nm. Le réacteur ne contient pas de composé actif (b3) surnageant, la dispersion est homogène. Le composé actif (b3) a été encapsulé par le copolymère (P1) en présence du copolymère (P2).

### Exemple 11 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids de pigment organique (noir de carbone Monarch 120 Cabot N° CAS 1333-86-4) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (±2°C) pendant 2 h puis refroidi à température ambiante.

On obtient une dispersion d'un composite selon l'invention comprenant un polymère (P1), un composé actif (b3) et un copolymère (P2). L'extrait sec final est de 23,6 % en poids et la taille des particules est répartie en trois populations : une majoritaire à 140 nm, une autre à 830 nm et une dernière à 4 400 nm. Le diamètre d₅₀, pour lequel au moins 50 % en volume des particules ont une taille inférieure à une certaine taille, est de 150 nm. Le réacteur ne contient pas de composé actif (b3) ayant précipité, la dispersion est homogène. Le composé actif (b3) a été encapsulé par le copolymère (P1) en présence du copolymère (P2).

### Exemple 12 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 6,02 g de 2-amino-2-méthylpropanol ;
- 58,6 g d'une composition B comprenant 88,0 % en poids de méthacrylate de butyle (b1), 4,5 % en poids de méthacrylate de stéaryle (b1), 1 % en poids d'azo-bis-isobutyronitrile (b2), 6 % en poids de pigment (silice cetyl silylate Aerosil R 816 Evonik N° CAS 199876-45-4) (b3) et 0,5 % en poids de réticulant diméthacrylate d'éthylène glycol (b6), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 24,0 % en poids et la taille des particules est répartie en deux populations : une minoritaire à 270 nm et une majoritaire à 6 600 nm. Le diamètre d₅₀, pour lequel au moins 50 % en volume des particules ont une taille inférieure à une certaine taille, est de 1 600 nm. Le réacteur ne contient pas de composé actif (b3) ayant précipité, la dispersion est homogène. Le composé actif (b3) a été encapsulé par le copolymère (P1) en présence du copolymère (P2).

### Exemple 13 :

Dans un réacteur agité à 200 tour/min, on ajoute à température ambiante :
- dans une composition A comprenant 135,3 g d'eau bipermutée et 87 g d'un copolymère (P2) de type HASE partiellement neutralisé et composé de 36,7 % en poids d'acide méthacrylique (a1), 27 % en poids d'acrylate d'éthyle (a2), 9,3 % en poids de méthacrylate d'iso-C₁₆OE₂₅ (a3 de formule (I) dans laquelle R¹ = C₁₆-alkyl ramifié, R² = méthacrylate, n = 0, m = 25) et de 27 % en poids de méthacrylate de méthyle puis neutralisé partiellement au moyen de 5,00 g de 2-amino-2-méthylpropanol ;
- 86,5 g d'une composition B comprenant 59,6 % en poids de méthacrylate de butyle (b1), 3,0 % en poids de méthacrylate de stéaryle (b1), 37,0 % en poids d'une huile d'origine végétale (huile de tournesol commerciale alimentaire Eco+) (b3) et 0,4 % en poids de réticulant diméthacrylate d'éthylène glycol (b6), puis
- 10,85 g d'une solution aqueuse de 4,4'-azobis(4-cyanopentanoic acid) (0,56 g) et 2-amino-2-méthylpropanol (0,29 g) (b2), puis
- une solution aqueuse d'acide gluconique (δ-gluconolactone N° CAS 90-80-2) à 5,4 % en poids, comprenant 4,85 g d'acide gluconique dans 85 g d'eau bipermutée.

Le réacteur est alors chauffé à 80°C (± 2°C) pendant 2 h puis refroidi à température ambiante.

On obtient une dispersion d'un composite selon l'invention comprenant un polymère (P1), un composé actif (b3) et un copolymère (P2). L'extrait sec final est de 28,5 % en poids et la taille des particules est répartie en trois populations : une majoritaire à 140 nm, une autre à 720 nm et une dernière à 3 800 nm. Le diamètre d₅₀, pour lequel au moins 50 % en volume des particules ont une taille inférieure à une certaine taille, est de 140 nm. Le réacteur ne contient pas de composé actif (b3) surnageant, la dispersion est homogène. Le composé actif (b3) a été encapsulé par le copolymère (P1) en présence du copolymère (P2).

## Revendications

1. Méthode de préparation dans l'eau d'un composite comprenant un polymère (P1) et au moins un composé actif, comprenant :
(a) la préparation à une température inférieure à 40°C d'un mélange comprenant de l'eau et au moins un copolymère (P2) de type HASE, totalement ou partiellement neutralisé au moyen d'une base ;
(b) l'addition sous agitation et à une température inférieure à 40°C :
(b1) d'au moins un monomère comprenant au moins une insaturation oléfinique polymérisable ;
(b2) d'au moins un composé générateur de radicaux ;
(b3) d'au moins un composé actif et
(c) la polymérisation sous agitation du monomère (b1) par chauffage à une température supérieure à 50°C.

2. Méthode selon la revendication 1 pour laquelle :
- des gouttelettes de monomère (b1) et de composé (b3) sont dispersées au moyen du copolymère (P2) de type HASE, de préférence des gouttelettes ayant une taille allant de 50 nm à 50 µm, préférentiellement de 50 nm à 500 nm ou de 1 µm à 50 µm ou bien de 50 nm à 1 µm ou encore de 500 nm à 50 µm ou
- des gouttelettes de polymère (P1) sont dispersées au moyen du copolymère (P2) de type HASE, de préférence des gouttelettes ayant une taille allant de 30 nm à 50 µm ou de 50 nm à 50 µm, préférentiellement de 30 nm à 500 nm ou de 50 nm à 500 nm ou de 1 µm à 50 µm ou bien de 30 nm à 1 µm ou de 50 nm à 1 µm ou encore de 500 nm à 50 µm ou
- l'émulsion de monomère (b1) ou de polymère (P1) préparée au moyen du copolymère (P2) de type HASE possède un extrait sec allant de 10 % en poids à 50 % en poids, de préférence de 15 % en poids à 45 % en poids ou de 20 % en poids à 40 % en poids d'émulsion.

3. Méthode selon l'une des revendications 1 ou 2 pour laquelle :
- la température des étapes (a) et (b), identique ou différente, est inférieure à 35°C ou inférieure à 25°C ou
- la température de l'étape (c) est supérieure à 60°C, de préférence supérieure à 70°C.

4. Méthode selon l'une des revendications 1 à 3 pour laquelle le polymère (P2) de type HASE est obtenu par réaction de polymérisation :
(a1) d'au moins un monomère anionique comprenant au moins une insaturation oléfinique polymérisable, de préférence un monomère anionique comprenant au moins une insaturation oléfinique polymérisable et au moins une fonction acide carboxylique, de préférence le monomère anionique est choisi parmi l'acide acrylique et l'acide méthacrylique, un sel d'acide acrylique, un sel d'acide méthacrylique,
(a2) d'au moins un ester d'un composé dérivé d'un acide choisi parmi l'acide acrylique, l'acide méthacrylique, l'acide itaconique et l'acide maléique, de préférence l'acide acrylique ou l'acide méthacrylique,
(a3) d'au moins un composé de formule (I) :
R¹-(OE)ₘ-(OP)ₙ-R² (I)
dans laquelle :
- m et n, identiques ou différents, représentent indépendamment 0 ou un nombre entier ou décimal inférieur à 150, m ou n est différent de 0,
- OE représente indépendamment un groupement CH₂CH₂O,
- OP représente indépendamment un groupement choisi parmi CH(CH₃)CH₂O et CH₂CH(CH₃)O,
- R¹ représente un groupement C₆-C₄₀-alkyle, linéaire ou ramifié, de préférence un groupement C₈-C₂₀-alkyle, linéaire ou ramifié et
- R² représente un groupement comprenant au moins une insaturation oléfinique polymérisable, de préférence un groupement acrylate ou un groupement méthacrylate, éventuellement
(a4) d'au moins un composé choisi parmi acide 2-acrylamido-2-méthylpropane sulfonique, acide éthoxyméthacrylate sulfonique, méthallyl sulfonate de sodium, styrène sulfonate et leurs sels et également éventuellement
(a5) d'au moins un monomère réticulant ou d'au moins un monomère comprenant au moins deux insaturations oléfiniques.

5. Méthode selon l'une des revendications 1 à 4 pour laquelle :
- le composé générateur de radicaux (b2), de préférence un composé générateur de radicaux (b2) lipophile, le monomère (b1) et de composé (b3) sont ajoutés sous la forme d'un mélange ou
- le composé générateur de radicaux (b2), de préférence un composé générateur de radicaux (b2) hydrophile, le monomère (b1) et le composé (b3) sont ajoutés séparément ou successivement, de préférence le composé générateur de radicaux (b2) étant alors ajouté après le monomère (b1) et le composé (b3).

6. Méthode selon l'une des revendications 1 à 5 pour laquelle le monomère (b1) est un monomère non ionique, de préférence un monomère non ionique comprenant au moins une insaturation oléfinique polymérisable, en particulier une insaturation éthylénique polymérisable et notamment une fonction vinylique polymérisable, plus préférentiellement un monomère non ionique choisi parmi styrène, vinylcaprolactam, N-isopropyl-acrylamide, N-vinyl-pyrrolidone, les esters d'un acide comprenant au moins une fonction acide monocarboxylique, en particulier un ester d'un acide choisi parmi l'acide acrylique, l'acide méthacrylique, un sel d'acide acrylique, un sel d'acide méthacrylique et leurs mélanges, par exemple acrylate d'alkyle, en particulier acrylate de C₁-C₂₄-alkyl, préférentiellement acrylate de C₁-C₂₀-alkyl ou acrylate de C₁-C₄-alkyl, plus préférentiellement acrylate de méthyle, acrylate d'éthyle, acrylate de propyle, acrylate d'isobutyle, acrylate de n-butyle, acrylate de stéaryle, méthacrylate d'alkyle, en particulier méthacrylate de C₁-C₂₄-alkyl, préférentiellement méthacrylate de C₁-C₂₀-alkyl ou méthacrylate de C₁-C₄-alkyl, plus préférentiellement méthacrylate de méthyle, méthacrylate d'éthyle, méthacrylate de propyle, méthacrylate d'isobutyle, méthacrylate de n-butyle, méthacrylate de stéaryle, acrylate d'aryle, de préférence benzylacrylate, phénoxyéthylacrylate, méthacrylate d'aryle, de préférence phénylméthacrylate, benzylméthacrylate, phénoxyéthylméthacrylate.

7. Méthode selon l'une des revendications 1 à 6 pour laquelle le composé générateur de radicaux (b2) est un composé choisi parmi un composé azoïque, de préférence azo-bis-isobutyronitrile (AZDN ou AIBN), un composé peroxyde, de préférence peroxyde de benzoyle, hydroperoxyde de benzoyle et leurs mélanges, persulfates de métaux alcalins, en particulier le persulfate de sodium, le persulfate de potassium, le persulfate d'ammonium, les peroxydes partiellement hydrosolubles, en particulier, l'hydroperoxyde de t-butyle, l'hydroperoxyde de cumyle, les persulfates associés à un ion ferreux, à un ion sulfite ou à un ion bisulfite et leurs mélanges, éventuellement associé à au moins un agent de transfert de polymérisation radicalaire contrôlée, notamment un agent de transfert de type Raft (*reversible addition-fragmentation chain transfer* ou polymérisation radicalaire contrôlée par transfert de chaîne réversible par addition-fragmentation), par exemple un dérivé de xanthate, de préférence dipropyl trithiocarbonate (DPTTC ou disodium 2,2'-(thiocarbonylbisthio)dipropanoate - n° CAS 864970-33-2), les composés mercaptans, en particulier les composés mercaptans comprenant au moins quatre atomes de carbone tels que le butylmercaptan, le n-octylmercaptan, le n-dodécylmercaptan, le *tert-*dodécylmercaptan, l'isooctyl 3-mercaptopropionate.

8. Méthode selon l'une des revendications 1 à 7 pour laquelle :
• le composé (b3) est un composé hydrophile ou un composé lipophile ou bien un composé non-ionique ou un composé apolaire ou bien encore
• le composé (b3) est choisi parmi un composé cosmétique, un composé de soin de la peau, un composé anti-âge, une vitamine, une co-enzyme, un peptide, un composé de soin des cheveux, un composé odorant, un composé aromatique, un composé pharmaceutique, un composé antiseptique, un composé phytosanitaire, un colorant, par exemple phtalocyanine, un pigment, du carbone, par exemple sous forme de nanotubes de carbone, un composé optiquement actif, un composé détergent, un composé adoucissant, un composé luminescent, un composé phosphorescent, un composé fluorescent, un composé métallescent, un composé perlescent, un composé opacifiant, notamment un composé ayant un indice de réfraction supérieur à 1,65 (dioxyde de titane, oxyde de zinc, dioxyde de zinc, alumine, nitrure de bore, talc, kaolin, mica, PbO, ZnSi, ZnSiO₄, ZnS, ZnSe₂), un composé opacifiant revêtu, un composé photochromique, un composé nacré, un composé plastifiant (triethyl citrate, tricaprylin, trilaurin, tripalmitin, triacetin, acetyltriethyl citrate, huile de paraffine), un composé acide gras (acide stearique, acide arachidique, acide palmitoleique, acide oleique, acide linoleique, acide linolaidique, acide arachidonique, acide myristoleique, acide erucique, stéarate de magnésium, oleate de magnésium, stéarate de calcium, linoleate de calcium, stéarate de sodium), un composé huileux, un filtre solaire minéral, un filtre solaire organique, un composé hydratant, un composé azurant optique, un composé blanchissant, un composé antibactérien, le menthol, le chèvrefeuille, l'huile de cananga, le citronellal, l'aurantiol, le limonène, les acides gras, les alcools gras, les beurres, les cires (par exemple les cires d'abeille), les huiles, de préférence une huile choisie parmi les huiles minérales (par exemple l'huile de paraffine, l'huile de vaseline, les huiles minérales ayant un point d'ébullition allant de 300 à 400°C), les huiles d'origine animale (par exemple les squalènes, le squalane, le perhydrosqualène), les huiles végétales (par exemple l'huile d'amande douce, l'huile de calophyllum, l'huile de palme, l'huile de noyau d'abricot, l'huile d'avocat, l'huile de jojoba, l'huile d'olive, l'huile de ricin, les huiles de germes de céréales comme l'huile de germe de blé, la fraction liquide de beurre de karité, l'huile de soja, l'huile de colza), octyl dodecanol, octyldodecyl neopentanoate, triglycéride caprylic/capric, pentaerythrityl tetraisostearate, isodecyl neopentanoate, diisopropyl sebacate, C₁₂-C₁₅ alkyl benzoate, ethylhexyl ethylhexanoate, 2-ethylhexyl hydroxystearate, les composés insaponifiables issus d'huiles naturelles, les huiles synthétiques (par exemple le polyisobutène hydrogéné, les esters d'acides gras tels que l'huile de purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, les esters dérivés d'acide lanolique tels que le lanolate de disopropyle, le lanolate d'isocétyle, les acétylglycérides, les octanoates d'alcool, les octanoates de polyalcools, les décanoates d'alcool, les décanoates de polyalcools, notamment les octanoates de glycol, les octanoates de glycérol, les décanoates de glycol, les décanoates de glycérol, les ricinoléates d'alcools, les ricinoléates de polyalcools), les terpènes, les polyterpènes, le phytostérols, les huiles siliconées (par exemple les cyclométhicones, les polydiméthylsiloxanes de faible poids moléculaire ou huiles de silicone, les polydiméthylsiloxanes de haut poids moléculaire ou gommes de silicone, les polyméthylsiloxanes, les diméthiconols, les polydiméthylsiloxanes phénylées, les siloxanols de faible poids moléculaire, les siloxanols de haut poids moléculaire, les triméthylsiloxysilicates), les huiles fluorées (par exemple les perfluoroéthers fluorés et les silicones fluorés) et leurs combinaisons ; de préférence
• le composé (b3) est choisi parmi les composés actifs cosmétiques, par exemple un composé optiquement actif, un composé de soin de la peau, un composé anti-âge, une vitamine, une co-enzyme, un peptide, un composé de soin des cheveux, un composé odorant, un composé aromatique.

9. Méthode selon l'une des revendications 1 à 8 pour laquelle l'étape (b) comprend également l'addition d'au moins un autre monomère, de préférence un monomère choisi parmi :
(b4) au moins un monomère anionique, de préférence un monomère anionique comprenant au moins une insaturation oléfinique polymérisable, en particulier une insaturation éthylénique polymérisable et notamment une fonction vinylique polymérisable, et au moins une fonction acide carboxylique, de préférence un monomère choisi parmi l'acide acrylique, l'acide méthacrylique, l'acide maléique, l'acide itaconique, l'acide crotonique, un sel d'acide acrylique, un sel d'acide méthacrylique, un sel d'acide maléique, un sel d'acide itaconique, un sel d'acide crotonique,
(b5) au moins un composé choisi parmi acide 2-acrylamido-2-méthylpropane sulfonique, acide éthoxyméthacrylate sulfonique, méthallyl sulfonate de sodium, styrène sulfonate et leurs sels,
(b6) au moins un monomère comprenant au moins deux insaturations oléfiniques,
(b7) au moins un monomère hydrophile non ionique, par exemple vinyl lactams comme N-vinylpyrrolidone, acrylates d'hydroxypropyle, hydroxy-C₁-C₅-alkyl-acrylate, hydroxy-C₁-C₅-alkyl-méthacrylate, acrylamides, méthacrylamides, diacetone-acrylamide, N-isopropylacrylamide.

10. Méthode selon l'une des revendications 1 à 9 mettant en oeuvre une quantité de composé tensioactif inférieure à 5 % en poids du poids de l'ensemble des monomères, préférentiellement moins de 3 % en poids ou moins de 1 % en poids du poids de l'ensemble des monomères ou ne mettant en oeuvre aucun composé tensioactif.

11. Composite comprenant au moins un polymère (P1), un copolymère (P2) de type HASE et au moins un composé actif (b3), susceptible d'être préparé selon la méthode selon l'une des revendications 1 à 9, de préférence pour lequel le composé actif (b3) est encapsulé par du polymère (P1).

12. Méthode de protection d'au moins un composé actif par encapsulation au sein d'un composite préparé selon la méthode défini selon l'une des revendications 1 à 9.

13. Méthode de traitement non-thérapeutique comprenant l'application d'au moins un composite défini selon la revendication 11, de préférence l'application sur un substrat, par exemple un substrat kératinique, en particulier les cheveux, les poils, les ongles et la peau.

14. Méthode de traitement non-thérapeutique selon la revendication 13 au cours de laquelle :
• le composé actif (b3) n'est pas libéré du composite, de préférence le composé actif (b3) n'est pas libéré du composite ; ou
• le composé actif (b3) est libéré de manière active ou est libéré de manière passive, notamment de manière progressive dans le temps, de préférence le composé actif (b3) est libéré de manière active ou est libéré de manière passive.

15. Méthode de traitement non-thérapeutique selon l'une des revendications 13 et 14 au cours de laquelle le composé actif (b3) est libéré au moyen du composite, de préférence libéré de manière contrôlée ou de manière progressive, par exemple libéré par un changement de pH, par un changement de force ionique, par application d'une contrainte physique ou par un changement de température.

## Patentansprüche

1. Verfahren zur Herstellung eines Verbundwerkstoffs aus einem Polymer (P1) und mindestens einem Wirkstoff in Wasser, umfassend:
(a) Herstellen, bei einer Temperatur von unter 40 °C, eines Gemischs aus Wasser und mindestens einem Copolymer (P2) vom Typ HASE, das vollständig oder teilweise mittels einer Base neutralisiert ist;
(b) Zugeben unter Rühren und bei einer Temperatur von unter 40 °C:
(b1) mindestens eines Monomers, das mindestens eine polymerisierbare olefinische Ungesättigtheit hat;
(b2) mindestens einer radikalbildenden Verbindung;
(b3) mindestens eines Wirkstoffs; und
(c) Polymerisieren unter Rühren des Monomers (b1) durch Erwärmen auf eine Temperatur von über 50 °C.

2. Verfahren nach Anspruch 1, wobei:
- Tröpfchen des Monomers (b1) und der Verbindung (b3) mittels Copolymer (P2) vom Typ HASE dispergiert werden, vorzugsweise Tröpfchen mit einer Größe von 50 nm bis 50 µm, noch mehr bevorzugt von 50 nm bis 500 nm bzw. von 1 µm bis 50 µm oder von 50 nm bis 1 µm oder von 500 nm bis 50 µm, oder
- Tröpfchen des Polymers (P1) mittels Copolymer (P2) vom Typ HASE dispergiert werden, vorzugsweise Tröpfchen mit einer Größe von 30 nm bis 50 µm oder von 50 nm bis 50 µm, noch mehr bevorzugt von 30 nm bis 500 nm oder von 50 nm bis 500 nm bzw. von 1 µm bis 50 µm oder von 30 nm bis 1 µm oder von 50 nm bis 1 µm oder von 500 nm bis 50 µm, oder
- die Emulsion von Monomer (b1) oder Polymer (P1), die mittels Copolymer (P2) vom Typ HASE hergestellt wird, einen Trockenextrakt von 10 bis 50 Gew.-%, vorzugsweise von 15 bis 45 Gew.-% oder von 20 bis 40 Gew.-% der Emulsionszusammensetzung hat.

3. Verfahren nach einem der Ansprüche 1 oder 2, wobei:
- die Temperatur der Schritte (a) und (b), die gleich oder unterschiedlich sein kann, unter 35 °C oder unter 25 °C liegt, oder
- die Temperatur des Schritts (c) über 60 °C, vorzugsweise über 70 °C, liegt.

4. Verfahren nach einem der Ansprüche 1 bis 3, wobei das Polymer (P2) vom Typ HASE durch eine Polymerisationsreaktion folgender Stoffe erhalten wird:
(a1) mindestens einem anionischen Monomer, das mindestens eine polymerisierbare olefinische Ungesättigtheit hat, vorzugsweise einem anionischen Monomer, das mindestens eine polymerisierbare olefinische Ungesättigtheit und mindestens eine Carbonsäure-Funktion aufweist, noch mehr bevorzugt einem anionischen Monomer, das ausgewählt ist aus Acrylsäure und Methacrylsäure, einem Salz der Acrylsäure, einem Salz der Methacrylsäure,
(a2) mindestens einem Ester einer von einer Säure abgeleiteten Verbindung, wobei diese Säure ausgewählt ist aus Acrylsäure, Methacrylsäure, Itaconsäure und Maleinsäure, vorzugsweise Acrylsäure oder Methacrylsäure,
(a3) mindestens einer Verbindung mit der Formel (I):
R¹-(OE)ₘ-(OP)ₙ-R² (I)
wobei:
- m und n, die gleich oder unterschiedlich sein können, unabhängig 0 oder eine Ganzzahl oder eine Dezimalzahl unter 150 darstellen, wobei m oder n ungleich 0 ist,
- OE unabhängig eine CH₂CH₂O-Gruppe darstellt,
- OP unabhängig eine Gruppe darstellt, die ausgewählt ist aus CH(CH₃)CH₂O und CH₂CH(CH₃)O,
- R¹ eine C₆-C₄₀-Alkyl-, Linear- oder Verzweigungsgruppe darstellt, vorzugsweise eine C₈-C₂₀-Alkyl-, Linear- oder Verzweigungsgruppe, und
- R² eine Gruppe mit mindestens einer polymerisierbaren olefinischen Ungesättigtheit darstellt, vorzugsweise eine Acrylat-Gruppe oder Methacrylat-Gruppe, eventuell
(a4) mindestens einer Verbindung, ausgewählt aus 2-Acrylamido-2-methylpropansulfonsäure, Ethoxymethacrylatsulfonsäure, Methallylnatriumsulfonat, Styrolsulfanat und deren Salzen sowie eventuell
(a5) mindestens einem vernetzenden Monomer oder mindestens einem Monomer, das mindestens zwei olefinische Ungesättigtheiten hat.

5. Verfahren nach einem der Ansprüche 1 bis 4, wobei:
- die radikalbildende Verbindung (b2), vorzugsweise eine lipophile radikalbildende Verbindung (b2), das Monomer (b1) und die Verbindung (b3) in Form eines Gemischs zugefügt werden, oder
- die radikalbildende Verbindung (b2), vorzugsweise eine hydrophile radikalbildende Verbindung (b2), das Monomer (b1) und die Verbindung (b3) getrennt oder nacheinander zugefügt werden, wobei die radikalbildende Verbindung (b2) vorzugsweise nach dem Monomer (b1) und der Verbindung (b3) zugesetzt wird.

6. Verfahren nach einem der Ansprüche 1 bis 5, wobei das Monomer (b1) ein nicht-ionisches Monomer ist, vorzugsweise ein nicht-ionisches Monomer mit mindestens einer polymerisierbaren olefinischen Ungesättigtheit, insbesondere einer polymerisierbaren ethylenischen Ungesättigtheit, und insbesondere einer polymerisierbaren funktionellen Vinylgruppe, noch mehr bevorzugt ein nicht-ionisches Monomer, ausgewählt aus Styrol, Vinylcaprolactam, N-Isopropylacrylamid, N-Vinylpyrrolidon, den Estern einer Säure mit mindestens einer Monocarbonsäurefunktion, insbesondere einem Ester einer Säure, ausgewählt aus Acrylsäure, Methacrylsäure, einem Salz der Acrylsäure, einem Salz der Methacrylsäure und deren Mischungen, beispielsweise Alkylacrylat, insbesondere C₁-C₂₄-Alkylacrylat, vorzugsweise C₁-C₂₀-Alkylacrylat oder C₁-C₄-Alkylacrylat, noch mehr bevorzugt Methylacrylat, Ethylacrylat, Propylacrylat, Isobutylacrylat, n-Butylacrylat, Stearylacrylat, Alkylmethacrylat, insbesondere C₁-C₂₄-Alkylmethacrylat, vorzugsweise C₁-C₂₀-Alkylmethacrylat oder C₁-C₄-Alkylmethacrylat, noch mehr bevorzugt Methylmethacrylat, Ethylmethacrylat, Propylmethacrylat, Isobutylmethacrylat, n-Butylmethacrylat, Stearylmethacrylat, Arylacrylat, vorzugsweise Benzylacrylat, Phenoxyethylacrylat, Arylmethacrylat, noch mehr bevorzugt Phenylmethacrylat, Benzylmethacrylat, Phenoxyethylmethacrylat.

7. Verfahren nach einem der Ansprüche 1 bis 6, wobei die radikalbildende Verbindung (b2) eine Verbindung ist, die ausgewählt ist aus einer Azoverbindung, vorzugsweise Azo-bis-isobutyronitril (AZDN oder AIBN), einer Peroxidverbindung, vorzugsweise Benzoylperoxyd, Benzoylhydroperoxyd und deren Mischungen, Alkalimetallpersulfaten, insbesondere Natriumpersulfat, Kaliumpersulfat, Ammoniumpersulfat, teilweise wasserlöslichen Peroxiden, insbesondere t-Butylhydroperoxid, Cumylhydroperoxyd, Persulfaten assoziiert mit einem Eisenion, einem Sulfition oder einem Bisulfition und deren Mischungen, eventuell in Verbindung mit mindestens einem Übertragungsmittel der kontrollierten radikalischen Polymerisation, insbesondere einem Übertragungsmittel vom Typ RAFT (*reversible Additions-Fragmentierungs-Kettenübertragung* oder Reversible Additions-Fragmentierungs-Kettenübertragung), beispielsweise einem Derivat von Xanthat, vorzugsweise Dipropyltrithiocarbonat (DPTTC oder Dinatrium-2,2'-(thiocarbonylbisthio)dipropanoat - CAS-Nr. 864970-33-2), insbesondere den Mercaptanverbindungen mit mindestens vier Kohlenstoffatomen, wie zum Beispiel Butylmercaptan, n-Octylmercaptan, n-Dodecylmercaptan, tert-Dodecylmercaptan, Isooctyl-3-mercaptopropionat.

8. Verfahren nach einem der Ansprüche 1 bis 7, wobei:
• die Verbindung (b3) eine hydrophile Verbindung, eine lipophile Verbindung, eine nicht-ionische Verbindung oder eine apolare Verbindung ist, oder
• die Verbindung (b3) ausgewählt ist aus einer kosmetischen Zusammensetzung, einer Hautpflegezusammensetzung, einer Anti-Aging-Verbindung, einem Vitamin, einem Coenzym, einem Peptid, einer Haarpflegezusammensetzung, einer Geruchsstoffverbindung, einer aromatischen Verbindung, einer pharmazeutischen Verbindung, einer antiseptischen Verbindung, einer Pflanzenschutz-Zusammensetzung, einem Farbstoff, beispielsweise Phthalocyanin, einem Pigment, Kohlenstoff, zum Beispiel in Form von Kohlenstoffnanoröhren, einer optisch aktiven Verbindung, einer Reinigungsmittelverbindung, einer Weichmacherverbindung, einer lumineszenten Verbindung, einer phosphoreszierenden Verbindung, einer fluoreszierenden Verbindung, einer metallisierenden Verbindung, einer Perlglanzverbindung, einer opalisierenden Verbindung, insbesondere einer Verbindung mit einem Brechungsindex von über 1,65 (Titandioxid, Zinkoxid, Zinkdioxid, Aluminiumoxid, Bornitrid, Talk, Kaolin, Mica, PbO, ZnSi, ZnSiO₄, ZnS, ZnSe₂), einer beschichteten opalisierenden Zusammensetzung, einer photochromen Zusammensetzung, einer Perlmuttverbindung, einer plastifizierenden Zusammensetzung (Triethylcitrat, Tricaprylin, Trilaurin, Tripalmitin, Triacetin, Acetyltriethylcitrat, Paraffinöl), einer Fettsäureverbindung (Stearinsäure, Arachidsäure, Palmitoleinsäure, Oleinsäure, Linolsäure, Linolensäure, Arachidonsäure, Myristoleinsäure, Erucasäure, Magnesiumstearat, Magnesiumoleat, Calciumstearat, Calciumlinoleat, Natriumstearat), einer öligen Verbindung, einem mineralischen Sonnenschutz, einem organischen Sonnenschutz, einer feuchtigkeitsspendenden Verbindung, einer optisch aufhellenden Verbindung, einer bleichenden Verbindung, einer antibakteriellen Verbindung, Menthol, Geißblatt, Canangaöl, Citronellal, Aurantiol, Limonen, Fettsäuren, Fettalkohole, Butter, Wachse (zum Beispiel Bienenwachs), Ölen, vorzugsweise einem Öl ausgewählt aus Mineralölen (z. B. Paraffinöl, Vaselineöl, Mineralölen mit einem Siedepunkt zwischen 300 und 400 °C), Ölen tierischen Ursprungs (z. B. Squalen, Squalan, Perhydrosqualen), pflanzlichen Ölen (z. B. Süßmandelöl, Calphyllumöl, Palmöl, Aprikosenkernöl, Avocadoöl, Jojobaöl, Olivenöl, Rizinusöl, Getreidekeimölen, wie Weizenkeimöl, flüssige Phase von Sheabutter, Sojaöl, Rapsöl), Octyldodecanol, Octyldodecylneopentanoat, Caprylic/Capric Triglyceride, Pentaerythrityltetraisostearat, Isodecylneopentanoat, Diisopropylsebacat, C₁₂-C₁₅-Alkylbenzoat, Ethylhexylethylhexanoat, 2-Ethylhexylhydroxystearat, unverseifbaren Verbindungen aus natürlichen Ölen, synthetischen Ölen (z. B. hydriertem Polyisobuten, Fettsäureester wie Bürzeldrüsenöl, Butylmyristat, Isopropylmyristat, Celylmyristat, Isopropylpamitat, Butylstearat, Hexadecylstearat, Isopropylstearat, Octylstearat, Isocetylstearat, Decyloleat, Hexyllaurat, Propylenglycoldicapryat, Ester der Lanolinsäure wie Disopropyllanolat, Isocetyllanolat, Acetylglyceriden, Alkoholoctanoaten, Polyalkoholoctanoaten, Alkoholdecanoaten, Polyalkoholdecanoaten, insbesondere Glykoloctanoaten, Glycerinoctanoaten, Glykoldecanoaten, Glycerindecanoaten, Alkoholricinoleaten, Polyalkoholricinoleaten), Terpenen, Polyterpenen, Phytosterinen, Silikonölen (z. B. Cyclomethiconen, Polydimethylsiloxanen mit geringer Molekülmasse oder Silikonölen, Polydimethylsiloxanen mit hoher Molekülmasse oder Silikongummi, Polymethylsiloxanen, Dimethiconolen, phenylierten Polydimethylsiloxanen, Siloxanolen mit geringer Molekülmasse, Siloxanolen mit hoher Molekülmasse, Trimethylsiloxysilicaten), fluorierten Ölen (z. B. fluoriertem Perfluoroether und Fluorsilikonen) und deren Kombinationen; auf bevorzugte Weise
• die Verbindung (b3) ausgewählt ist aus den kosmetischen Wirkstoffen, zum Beispiel einer optisch aktiven Verbindung, einer Hautpflegezusammensetzung, einer Anti-Aging-Verbindung, einem Vitamin, einem Coenzym, einem Peptid, einer Haarpflegezusammensetzung, einer Geruchsstoffverbindung, einer aromatischen Verbindung.

9. Verfahren nach einem der Ansprüche 1 bis 8, wobei Schritt (b) ebenfalls die Zugabe mindestens eines anderen Monomers umfasst, vorzugsweise eines Monomers ausgewählt aus:
(b4) mindestens einem anionischen Monomer, vorzugsweise einem anionischen Monomer mit mindestens einer polymerisierbaren olefinischen Ungesättigtheit, insbesondere einer polymerisierbaren ethylenischen Ungesättigtheit, und insbesondere einer polymerisierbaren funktionellen Vinylgruppe, und mindestens einer Carbonsäure-Funktion, vorzugsweise einem Monomer ausgewählt aus Acrylsäure, Methacrylsäure, Maleinsäure, Itaconsäure, Crotonsäure, einem Salz der Acrylsäure, einem Salz der Methacrylsäure, einem Salz der Maleinsäure, einem Salz der Itaconsäure, einem Salz der Crotonsäure,
(b5) mindestens einer Verbindung ausgewählt aus 2-Acrylamido-2-methylpropansulfonsäure, Ethoxymethacrylatsulfonsäure, Methallylnatriumsulfonat, Styrolsulfanat und deren Salzen,
(b6) mindestens einem Monomer mit mindestens zwei olefinischen Ungesättigtheiten,
(b7) mindestens einem nicht-ionischen hydrophilen Monomer, beispielsweise Vinyllactamen wie N-Vinylpyrrolidon, Hydroxypropylacrylaten, C₁-C₅-Hydroxyalkylacrylat, C₁-C₅-Hydroxyalkylmethacryalt, Acrylamiden, Methacrylamiden, Diacetonacrylamid, N-Isopropylacrylamid.

10. Verfahren nach einem der Ansprüche 1 bis 9, wobei eine Menge an oberflächenaktiver Verbindung von weniger als 5 Gew.-% des Anteils aller Monomere, vorzugsweise weniger als 3 Gew.-% oder weniger als 1 Gew.-% des Anteils aller Monomere, verwendet wird oder wobei keine oberflächenaktive Verbindung eingesetzt wird.

11. Verbundwerkstoff, bestehend aus mindestens einem Polymer (P1), einem Copolymer (P2) vom Typ HASE und mindestens einem Wirkstoff (b3), der nach dem Verfahren laut einem der Ansprüche 1 bis 9 hergestellt werden kann, wobei der Wirkstoff (b3) vorzugsweise mit einem Polymer (P1) verkapselt ist.

12. Verfahren zum Schutz mindestens eines Wirkstoffs durch Verkapselung in einem Verbundwerkstoff, der nach dem Verfahren laut einem der Ansprüche 1 bis 9 hergestellt wird.

13. Verfahren zur nicht therapeutischen Behandlung mit Anwendung mindestens eines Verbundwerkstoffs nach Anspruch 11, vorzugsweise mit Anwendung auf einem Substrat, beispielsweise einem Keratinsubstrat, insbesondere Kopf- und Körperhaare, Nägel und Haut.

14. Verfahren zur nicht therapeutische Behandlung nach Anspruch 13, wobei:
• der Wirkstoff (b3) nicht aus dem Verbundwerkstoff freigesetzt wird ; oder
• der Wirkstoff (b3) aktiv oder passiv freigesetzt wird, insbesondere schrittweise im Laufe der Zeit, wobei vorzugsweise der Wirkstoff (b3) aktiv oder passiv freigesetzt wird.

15. Verfahren zur nicht therapeutischen Behandlung nach einem der Ansprüche 13 und 14, wobei der Wirkstoff (b3) mithilfe des Verbundwerkstoffs freigesetzt wird, vorzugsweise kontrolliert oder schrittweise freigesetzt wird, beispielsweise durch eine Änderung des pH-Werts, eine Änderung der Ionenstärke, durch Anwendung einer physischen Belastung oder durch eine Temperaturänderung.

## Claims

1. A method for preparing in water a composite comprising a polymer (P1) and at least one active compound, comprising:
(a) the preparation, at a temperature below 40°C, of a mixture comprising water and at least one HASE-type copolymer (P2), completely or partially neutralised by means of a base;
(b) the addition under stirring and at a temperature below 40°C:
(b1) of at least one monomer comprising at least one polymerisable olefinic unsaturation;
(b2) of at least one radical-generating compound;
(b3) of at least one active compound and
(c) the polymerisation under stirring of monomer (b1) by heating to a temperature above 50°C.

2. The method according to claim 1, in which:
- droplets of monomer (b1) and of compound (b3) are dispersed by means of the HASE copolymer (P2), preferably droplets with a size ranging from 50 nm to 50 µm, preferentially from 50 nm to 500 nm or from 1 µm to 50 µm or from 50 nm to 1 µm or from 500 nm to 50 µm or
- droplets of polymer (P1) are dispersed by means of the HASE copolymer (P2), preferably droplets with a size ranging from 30 nm to 50 µm or from 50 nm to 50 µm, preferentially from 30 nm to 500 nm or from 50 nm to 500 nm or from 1 µm to 50 µm or from 30 nm to 1 µm or from 50 nm to 1 µm or from 500 nm to 50 µm or
- the emulsion of monomer (b1) or of polymer (P1) prepared using the HASE copolymer (P2) has a solids content ranging from 10% by weight to 50% by weight, preferably from 15% by weight to 45% by weight or from 20% by weight to 40% by weight of emulsion.

3. The method according to one of claims 1 or 2, in which:
- the temperature in steps (a) and (b), identical or different, is below 35°C or below 25°C or
- the temperature in step (c) is above 60°C, preferably above 70°C.

4. The method according to one of claims 1 to 3, in which the HASE polymer (P2) is obtained by polymerisation reaction:
(a1) of at least one anionic monomer comprising at least one polymerisable olefinic unsaturation, preferably an anionic monomer comprising at least one polymerisable olefinic unsaturation and at least one carboxylic acid group; preferably, the anionic monomer is chosen among acrylic acid and methacrylic acid, an acrylic acid salt, a methacrylic acid salt,
(a2) of at least one ester from a compound derived from an acid chosen among acrylic acid, methacrylic acid, itaconic acid and maleic acid, preferably acrylic acid or methacrylic acid,
(a3) of at least one compound of formula (I):
R¹-(EO)ₘ-(PO)ₙ-R² (I)
wherein:
- m and n, identical or different, independently represent 0 or an integer or decimal less than 150, m or n is different from 0,
- EO independently represents a CH₂CH₂O group,
- PO independently represents a group chosen among CH(CH₃)CH₂O and CH₂CH(CH₃)O,
- R¹ represents a straight or branched C₆-C₄₀-alkyl group, preferably a straight or branched C₈-C₂₀-alkyl group and
- R² represents a group comprising at least one polymerisable olefinic unsaturation, preferably an acrylate group or a methacrylate group, optionally
(a4) of at least one compound chosen among 2-acrylamido-2-methylpropane sulphonic acid, ethoxy methacrylate sulphonic acid, sodium methallyl sulphonate, styrene sulphonate and salts thereof and also optionally
(a5) of at least one cross-linking monomer or of at least one monomer comprising at least two olefinic unsaturations.

5. The method according to one of claims 1 to 4, in which:
- radical-generating compound (b2), preferably a lipophilic radical-generating compound (b2), monomer (b1) and compound (b3) are added as a mixture or
- radical-generating compound (b2), preferably a hydrophilic radical-generating compound (b2), monomer (b1) and compound (b3) are added separately or successively; preferably radical-generating compound (b2) is then added after monomer (b1) and compound (b3).

6. The method according to one of claims 1 to 5, in which monomer (b1) is a non-ionic monomer, preferably a non-ionic monomer comprising at least one polymerisable olefinic unsaturation, in particular a polymerisable ethylenic unsaturation and particularly a polymerisable vinyl group, more preferentially a non-ionic monomer chosen among styrene, vinylcaprolactam, N-isopropylacrylamide, N-vinylpyrrolidone, esters of an acid comprising at least one monocarboxylic acid group, particularly an ester of an acid chosen among acrylic acid, methacrylic acid, an acrylic acid salt, a methacrylic acid salt and mixtures thereof, for example alkyl acrylate, particularly C₁-C₂₄-alkyl acrylate, preferentially C₁-C₂₀-alkyl acrylate or C₁-C₄-alkyl acrylate, more preferentially methyl acrylate, ethyl acrylate, propyl acrylate, isobutyl acrylate, n-butyl acrylate, stearyl acrylate, alkyl methacrylate, in particular C₁-C₂₄ alkyl methacrylate, preferentially C₁-C₂₀ alkyl methacrylate or C₁-C₄ alkyl methacrylate, more preferentially methyl methacrylate, ethyl methacrylate, propyl methacrylate, isobutyl methacrylate, n-butyl methacrylate, stearyl methacrylate, aryl acrylate, preferably benzyl acrylate, phenoxyethyl acrylate, aryl methacrylate, preferably phenyl methacrylate, benzyl methacrylate, phenoxyethyl methacrylate.

7. The method according to one of claims 1 to 6, in which radical-generating compound (b2) is a compound chosen among an azo compound, preferably azo-bis-isobutyronitrile (AZDN or AIBN), a peroxide compound, preferably benzoyl peroxide, benzoyl hydroperoxide and mixtures thereof, alkaline metal persulphates, in particular sodium persulphate, potassium persulphate, ammonium persulphate, the partially water-soluble peroxides, in particular, t-butyl hydroperoxide, cumyl hydroperoxide, persulphates associated with a ferrous ion, a sulphite ion or a bisulphite ion and mixtures thereof, optionally associated with at least one controlled radical polymerisation transfer agent, in particular a RAFT (*reversible addition-fragmentation chain transfer*) transfer agent, for example a xanthate derivative, preferably dipropyl trithiocarbonate (DPTTC or disodium 2,2'-(thiocarbonylbisthio)dipropanoate - CAS No. 864970-33-2), mercaptan compounds, in particular mercaptan compounds comprising at least four carbon atoms such as butylmercaptan, n-octylmercaptan, n-dodecylmercaptan, tert-dodecylmercaptan, isooctyl 3-mercaptopropionate.

8. The method according to one of claims 1 to 7, in which:
• compound (b3) is a hydrophilic compound or a lipophilic compound or a non-ionic compound or a nonpolar compound or even
• compound (b3) is chosen among a cosmetic compound, a skin care compound, an anti-ageing compound, a vitamin, a co-enzyme, a peptide, a hair care compound, an odorous compound, an aromatic compound, a pharmaceutical compound, an antiseptic compound, a phytosanitary compound, a colorant, for example phthalocyanine, a pigment, carbon, for example in the form of carbon nanotubes, an optically active compound, a detergent compound, a softening compound, a luminescent compound, a phosphorescent compound, a fluorescent compound, a metallescent compound, a pearlescent compound, an opacifying compound, in particular a compound with a refractive index greater than 1.65 (titanium dioxide, zinc oxide, zinc dioxide, alumina, boron nitride, talc, kaolin, mica, PbO, ZnSi, ZnSiO₄, ZnS, ZnSe₂), a coated opacifying compound, a photochromic compound, a pearly compound, a plasticising compound (triethyl citrate, tricaprylin, trilaurin, tripalmitin, triacetin, acetyltriethyl citrate, paraffin oil), a fatty acid compound (stearic acid, arachidic acid, palmitoleic acid, oleic acid, linoleic acid, linolaidic acid, arachidonic acid, myristoleic acid, erucic acid, magnesium stearate, magnesium oleate, calcium stearate, calcium linoleate, sodium stearate), an oily compound, a mineral sunscreen, an organic sunscreen, a moisturising compound, an optical brightening compound, a whitening compound, an antibacterial compound, menthol, honeysuckle, cananga oil, citronellal, aurantiol, limonene, fatty acids, fatty alcohols, butters, waxes (for example beeswaxes), oils, preferably an oil chosen among mineral oils (for example paraffin oil, vaseline oil, mineral oils with a boiling point ranging from 300 to 400°C), oils of animal origin (for example squalene, squalane, perhydrosqualene), vegetable oils (for example sweet almond oil, calophyllum oil, palm oil, apricot kernel oil, avocado oil, jojoba oil, olive oil, castor oil, cereal germ oils such as wheat germ oil, shea butter liquid fraction, soybean oil, rapeseed oil), octyl dodecanol, octyldodecyl neopentanoate, caprylic/capric triglyceride, pentaerythrityl tetraisostearate, isodecyl neopentanoate, diisopropyl sebacate, C₁₂-C₁₅ alkyl benzoate, ethylhexyl ethylhexanoate, 2-ethylhexyl hydroxystearate, unsaponifiable compounds derived from natural oils, synthetic oils (for example hydrogenated polyisobutene, fatty acid esters such as purcellin oil, butyl myristate, isopropyl myristate, cetyl myristate, isopropyl palmitate, butyl stearate, hexadecyl stearate, isopropyl stearate, octyl stearate, isocetyl stearate, decyl oleate, hexyl laurate, propylene glycol dicaprylate, esters derived from lanolic acid such as disopropyl lanolate, isocetyl lanolate, acetylglycerides, alcohol octanoates, polyalcohol octanoates, alcohol decanoates, polyalcohol decanoates, in particular glycol octanoates, glycerol octanoates, glycol decanoates, glycerol decanoates, alcohol ricinoleates, polyalcohol ricinoleates), terpenes, polyterpenes, phytosterols, silicone oils (for example cyclomethicones, low molecular weight polydimethylsiloxanes or silicone oils, high molecular weight polydimethylsiloxanes or silicone gums, polymethylsiloxanes, dimethiconols, phenylated polydimethylsiloxanes, low molecular weight siloxanols, high molecular weight siloxanols, trimethylsiloxysilicates), fluorinated oils (for example fluorinated perfluoroethers and fluorinated silicones) and combinations thereof; preferably
• compound (b3) is chosen among cosmetic active compounds, for example an optically active compound, a skin care compound, an anti-ageing compound, a vitamin, a co-enzyme, a peptide, a hair care compound, an odorous compound, an aromatic compound.

9. The method according to one of claims 1 to 8, in which step (b) also comprises the addition of at least one other monomer, preferably a monomer chosen among:
(b4) at least one anionic monomer, preferably an anionic monomer comprising at least one polymerisable olefinic unsaturation, in particular a polymerisable ethylenic unsaturation and particularly a polymerisable vinyl group, and at least one carboxylic acid group, preferably a monomer chosen among acrylic acid, methacrylic acid, maleic acid, itaconic acid, crotonic acid, an acrylic acid salt, a methacrylic acid salt, a maleic acid salt, an itaconic acid salt, a crotonic acid salt,
(a5) at least one compound chosen among 2-acrylamido-2-methylpropane sulphonic acid, ethoxy methacrylate sulphonic acid, sodium methallyl sulphonate, styrene sulphonate and salts thereof,
(b6) at least one monomer comprising at least two olefinic unsaturations,
(b7) at least one non-ionic hydrophilic monomer, for example vinyl lactams such as N-vinylpyrrolidone, hydroxypropyl acrylates, hydroxy-Ci-Cs-alkyl-acrylate, hydroxy-C₁-C₅-alkyl-methacrylate, acrylamides, methacrylamides, diacetone-acrylamide, N-isopropylacrylamide.

10. The method according to one of claims 1 to 9 using an amount of surfactant compound of less than 5% by weight of the weight of all the monomers, preferentially less than 3% by weight or less than 1% by weight of the weight of all the monomers or not using any surfactant compounds.

11. A composite comprising at least one polymer (P1), a HASE copolymer (P2) and at least one active compound (b3), which can be prepared according to the method according to one of claims 1 to 9, preferably in which the active compound (b3) is encapsulated by polymer (P1).

12. A method for protecting at least one active compound by encapsulation within a composite prepared according to the method defined according to one of claims 1 to 9.

13. A non-therapeutic treatment method comprising the application of at least one composite defined according to claim 11, preferably, the application to a substrate, for example a keratin substrate, in particularly hair, nails and skin.

14. The non-therapeutic treatment method according to claim 13, during which:
• the active compound (b3) is not released from the composite; or
• the active compound (b3) is actively released or is passively released, in particular gradually over time, preferably the active compound (b3) is actively released or is passively released.

15. The non-therapeutic treatment method according to one of claims 13 and 14, during which the active compound (b3) is released by means of the composite, preferably released in a controlled manner or in a gradual manner, for example released by a pH change, by a change in ionic strength, by application of a physical stress or by a change in temperature.
